(19)

Europäisches
Patentamt
European
Patent Office
Office européen
des brevets

(11)     **EP 4 227 699 A1**

(12)                                    **EUROPEAN PATENT APPLICATION**

(43) Date of publication:
  **16.08.2023  Bulletin 2023/33**

(21) Application number: **23170840.5**

(22) Date of filing: **03.02.2021**

(51) International Patent Classification (IPC):
  **G01R 33/28** (2006.01)     **G01R 33/34** (2006.01)
  **H01Q 15/00** (2006.01)

(52) Cooperative Patent Classification (CPC):
  **G01R 33/36; H01Q 15/006;** G01R 33/288;
  G01R 33/34; H01Q 15/0086

(84) Designated Contracting States:
  **AL AT BE BG CH CY CZ DE DK EE ES FI FR GB
  GR HR HU IE IS IT LI LT LU LV MC MK MT NL NO
  PL PT RO RS SE SI SK SM TR**

(30) Priority:  **05.02.2020  GB 202001568
          10.07.2020  GB 202010705**

(62) Document number(s) of the earlier application(s) in
  accordance with Art. 76 EPC:
  **21704906.3 / 4 100 754**

(71) Applicant: **Medical Wireless Sensing Ltd.
  42 New Road
  London
  E1 2AX (GB)**

(72) Inventors:
  • **SAHA, Shimul**
   **London, E1 2AX (GB)**
  • **KOUTSOUPIDOU, Maria**
   **London, E1 2AX (GB)**
  • **KALLOS, Efthymios**
   **London, E1 2AX (GB)**

(74) Representative: **Cobbold, Alistair John et al
  Kilburn & Strode LLP
  Lacon London
  84 Theobalds Road
  London WC1X 8NL (GB)**

Remarks:
  This application was filed on 28-04-2023 as a
  divisional application to the application mentioned
  under INID code 62.

(54)   **DEVICES FOR MANIPULATING ELECTROMAGNETIC FIELDS IN A MAGNETIC RESONANCE
    SYSTEM**

(57)     There is provided a method of producing a device (10, 20) for manipulating a magnetic field of RF radiation from one or more RF antenna (53) in an MR system (50). The method comprises: determining a target resonant RF frequency of the device (10, 20) based on at least one characteristic of the one or more RF antenna (53); determining a design of the device (10, 20) to provide the device (10, 20) with the determined target resonant RF frequency; and making the device (10, 20) in accordance with the design. The device (10, 20) comprises: a plurality of conductive elements (12, 22) arranged in an array (14), wherein the array is arranged to redistribute energy between electric and magnetic fields of the RF radiation at a resonant RF frequency when receiving the RF radiation, the RF radiation having an RF wavelength greater than a respective dimension of each conductive element (12, 22); and a dielectric material (16, 26), wherein the dielectric material (16, 26) has a dielectric permittivity and a loss coefficient.

Fig. 5

## Description

FIELD

The present disclosure relates to devices and methods for manipulating the magnetic field of signals in a Magnetic Resonance (MR) system, MR systems including such devices or implementing such methods, and methods of producing such devices and systems.

BACKGROUND

Magnetic Resonance Imaging (MRI) is a widely used medical imaging method capable of measuring brain neural activity, detecting early cancerous cells, imaging nanoscale biological structures, controlling fluid dynamics and functional cardiovascular imaging.

The demand for MRI scans is increasing steadily, resulting in longer waiting times. Increasing demand for higher resolution imaging has led to the development of higher static magnetic field scanners (3T or higher). As the need for higher quality images and the volume of MRI scans are steadily increasing over time, national health systems experience high pressure in their effort to reduce waiting lists within existing facilities, resources, and budget constraints. Therefore, improvements in MRI screening efficiency under these conditions are needed to advance of medical imaging and diagnostics.

PCT application published as WO2017007365 (12 January 2017) describes a metamaterial device for improving the Signal-to-noise ratio (SNR) of Radio-Frequency (RF) signals and reducing Specific absorption rate (SAR) in an MRI system. The device, functioning as an electromagnetic field concentrator, produces local redistribution of radio-frequency fields close to the subject being examined. This is by virtue of the fact that the length of each conductor in the electromagnetic field concentrator satisfies the requirement for the emergence of half-wave resonance. This device is particularly suited for relatively low power MRI scans. Given the potential for significant concentration of electro-magnetic (EM) fields offered by this device, there is a risk of RF signals being concentrated to an unacceptable level of SAR in high power scans. A further problem is that, when the object being imaged is inside an MRI system, the dielectric properties of the object may detune a transmit or receive coil of the MRI system. It is also possible for the presence of the resonating the device to disrupt the functioning of the MRI system.

SUMMARY

According to the present disclosure, there is provided a method of producing a device for manipulating a magnetic field of RF radiation from one or more RF antenna in an MR system. The method comprises: determining a target resonance quality factor of the device based on at least one characteristic of the one or more RF antenna; determining a design of the device to provide the device with the determined target resonance quality factor; and making the device in accordance with the design. The device comprises: a plurality of conductive elements arranged in an array, wherein the array is arranged to redistribute energy between electric and magnetic fields of the RF radiation at a resonant RF frequency when receiving the RF radiation, the RF radiation having an RF wavelength greater than a respective dimension of each conductive element; and a dielectric material, wherein the dielectric material has a dielectric permittivity and a loss tangent.

According to the present disclosure, there is provided a method of producing a device for manipulating a magnetic field of RF radiation from one or more RF antenna in an MR system. The method comprises: determining a target resonant RF frequency of the device based on at least one characteristic of the one or more RF antenna; determining a design of the device to provide the device with the determined target resonant RF frequency; and making the device in accordance with the design. The device comprises: a plurality of conductive elements arranged in an array, wherein the array is arranged to redistribute energy between electric and magnetic fields of the RF radiation at a resonant RF frequency when receiving the RF radiation, the RF radiation having an RF wavelength greater than a respective dimension of each conductive element; and a dielectric material, wherein the dielectric material has a dielectric permittivity and a loss tangent.

The term "RF antenna" is primarily used herein but alternatively may be referred to as an RF coil or an RF element. In particular, "RF antenna" should be understood to refer to any component arranged to transmit and/or receive RF radiation in an MR system, such as an RF coil or an RF element.

The above methods may determine both a target resonant RF frequency and a target resonance quality factor of the device based on at least one characteristic of the one or more RF antenna and determine the design of the device to provide the device with both the determined target resonant RF frequency and the determined target resonance quality factor. The target resonant RF frequency and/or target resonance quality factor are determined such that the device does not detune the one or more RF antenna, or otherwise allows or optimises the MR system to operate and obtain images, when the device is used in the MR system.

The making the device in accordance with the design may produce the device having a resonance quality factor and/or resonant RF frequency equal to, or substantially equal to, the respective target resonance quality factor and/or target resonant RF frequency.

The determing a target resonance quality factor and/or determing a target resonant RF frequency may include receiving, at a processor, a user input defining the at least one characteristic of the one or more RF antenna. The determining may include retrieving from a look-up table the target resonance quality factor based on at least one characteristic of the one or more RF antenna. As an example the at least one characteristic of the one or more RF antenna may be provided by the user input. Alternatively or additionally, the determining may include measuring the at least one characteristic of the one or more RF antenna using a sensor.

The determining a target resonance quality factor and/or determining a target resonant RF frequency may include simulating, at a processor, one or more possible devices having respective resonance quality factors and the one or more RF antenna having the at least one characteristic. The target resonance quality factor and/or target resonant RF frequency may be determined based on the simulation. Alternatively or additionally, the determining may include calculating, at a processor, the target resonance quality factor and/or target resonant RF frequency using an equation based on the at least one characteristic of the one or more RF antenna.

The determining the design of the device to provide the device with the determined target resonance quality factor and/or determined target resonant RF frequency may include retrieving from a look-up table a design which provides the respective target resonance quality factor and/or determined target resonant RF frequency. Alternatively or additionally, the determining the design may include simulating, at a processor, one or more designs and determing which design provides a resonance quality factor and/or resonant RF frequency closest to the target resonance quality factor and/or the target resonant RF frequency. Alternatively or additionally, the determining the design may include making a one or more preliminary devices according to different designs and measuring the resonance quality factor and/or resonant RF frequency of the preliminary devices to determine which design provides a resonance quality factor and/or resonant RF frequency closest to the respective target resonance quality factor and/or the target resonant RF frequency.

The design of the device in any of the above methods may include one or more of: a positioning of the dielectric material, e.g. a positioning of the dielectric material relative to the plurality of conductive elements; the dielectric permittivity; the loss tangent; and one or more electronic components connected between two respective portions of the conductive elements. If the design of the device includes the one or more electronic components, the device may comprise a controller configured to control the one or more electronic components.

The design of the device, to provide the device with either the determined target resonance quality factor and/or the determined target resonant RF frequency, may include one of the following parameters: a positioning of the dielectric material, e.g. a positioning of the dielectric material relative to the plurality of conductive elements; the dielectric permittivity; the loss tangent; and one or more electronic components connected between two respective portions of the conductive elements. The design may comprise any combination of 2 or 3 of these parameters, or all four parameters. The design may also include additional parameters which affect either a quality factor and/or the resonant RF frequency of the device.

Each of the conductive elements may be elongate, wherein the dielectric material is located along a portion of each end of the conductive elements, wherein the length of the portions is selected based on the at least one characteristic of the RF antenna. A conductive element may be described as elongate if it has a length at least twice as long as the width or depth of the conductive element. Alternatively, elongate may refer to conductive elements having a length at least ten times as long as the width or depth of the conductive element. The portions of each end of each conductive element can also be referred to as distal portions, being distal relative to the centre point of the respective conductive element.

Each conductive element may have a length of approximately half the wavelength of the resonant RF frequency or the target resonant RF frequency. The conductive elements may be arranged substantially parallel to each other. The conductive elements may be non-magnetic.

The length of the portion of each end of the conductive elements along which the dielectric material is located may be determined based on the at least one characteristic of the one or more RF antenna.

The device may be arranged such that the length of the portion of each end of the conductive elements along which the dielectric material is located can be varied. In other words, the positioning of the dielectric material relative to the plurality of conductive elements is controllable, even after the device has been made. For example, the device may be arranged such that the dielectric material is slidable in direction along the length of the conductive elements.

The at least one characteristic of the one or more RF antenna may include one or more of: an RF frequency that the one or more RF antenna is arranged to transmit or receive; a resonance quality factor of the one or more RF antenna; and an object to be imaged by the one or more RF antenna. For example, the at least one characterisitc may correspond to a first RF antenna of the one or more RF antenna, and include one or more of the RF frequency, resonance quality factor and/or obejct to be imaged of the first RF antenna. The at least one characteristic may correspond to a plurality of RF antennas configured to produce RF radiation for the MR system. The least one characteristic may be a plurality of characteristics associated with the one or more RF antenna, wherein the plurality of characteristics may characterise first RF antenna of the one or more RF antenna, or may characterise all of the plurality of RF antennas, or different characteristics may characterise different RF anteannas of the plurality of RF antennas.

The loss tangent of the dielectric material may be determined based on the resonance quality factor of the one or more RF antenna such that the resonance quality factor of the device means the device does not detune the one or more RF antenna when used in the MR system. For example, this may be done by the loss tangent of the dielectric material being determined based on the resonance quality factor of the one or more RF antenna such that the resonance quality factor of the device is less than the resonance quality factor of the one or more RF antenna. The loss tangent of the dielectric material may be determined in order to produce the target resonance quality factor of the device, or in order to produce the target resonant RF frequency, or both.

The loss tangent of the dielectric material may be determined based on resonance quality factors of a plurality of RF antennas such that the resonance quality factor of the array is less than a lowest value of the resonance quality factors of the plurality of RF antennas.

The device may be a first device and the method(s) above may further comprise: making a second device in accordance with the design; and assembling the first device and the second device on either side of one or more spacer located at at least one end of the conductive elements to provide an imaging region between the first device and second device, wherein the imaging region may be for receiving an object to be imaged.

The device may be configured to concentrate a magnetic field of RF radiation from the one or more RF antenna when in the MR system. For example, in use, the device may increase the magentic field at an object to be imaged in order to improve resolution of imaging in the MR system.

According to the present disclosure, there is provided a kit comprising one or more RF antenna having at least one characteristic and a device for manipulating a magnetic field of RF signals in an MR system. The device comprises a plurality of conductive elements arranged in an array, wherein the array is arranged to redistribute energy between electric and magnetic fields of the RF radiation at a resonant RF frequency when receiving the RF radiation, the RF radiation having an RF wavelength greater than a respective dimension of each conductive element. The device comprises a dielectric material, wherein the dielectric material has a dielectric permittivity and a loss tangent. The device is arranged to resonate with a resonance quality factor and/or the resonant RF frequency based on the at least one characteristic of the one or more RF antenna.

The at least one characteristic may be a resonance quality factor of the one or more RF antenna and the resonance quality factor of the device is less than the resonance quality factor of the one or more RF antenna.

The device of the kit may be made using any of the methods described above, and have any of the device features described above.

According to the present disclosure, there is provided an MR system comprising an imaging region arranged to receive an object to be imaged; a magnetic field generator arranged to produce a static magnetic field in the imaging region; a kit as described above, wherein the one or more RF antenna is arranged to irradiate the object with the RF radiation; and an RF receiver arranged to receive a return RF signal from the object for imaging the object; wherein a device as described above is arranged between the imaging region and either the one or more RF antenna or the RF receiver, or both. The one or more RF receiver may be the one or more RF antenna.

The device according the methods, kits and systems described above may include any of the following features.

The dielectric material may be ceramic. Additionally or alternatively, the dielectric material may comprise at least one of: a powder; a mixture of powders; and/or a slurry, wherein the slurry is a mixture of water and one or more powder, The dielectric permittivity may be greater than 50, optionally, greater than 100, wherein the dielectric permittivity is relative dielectric permittivity. The plurality of conductive elements may be a plurality of metal strips on a printed circuit board (PCB).

The dielectric permittivity, as referred to throughout the disclosure, in general refers to a relative dielectric permittivity.

The loss tangent of the dielectric material, as referred to throughout the disclosure, may be the loss tangent of the dielectric material at, or substantially at, the resonant RF frequency or the target resonant RF frequency. The loss tangent may be an average loss tangent of the dielectric material in a bandwidth containing the resonant RF frequency or the target resonant RF frequency. The loss tangent may be characterised by a series of values of loss tangent each at a respective RF frequency. The loss tangent is an example of a loss coefficient of dielectric material. In each of the described arrangements herein, an alternative loss coefficient could be used instead of the loss tangent.

BRIEF SUMMARY OF THE FIGURES

Specific embodiments are now described by way of example and with reference to the accompanying drawings, in which:

Figure 1 shows an isometric view of a device for manipulating magnetic fields of RF signals in an MR system;
Figure 2 shows an isometric view of a device for manipulating magnetic fields of RF signals in an MR system;
Figure 3 shows a circuit diagram of an electronic device connected between portions of conductive elements of a device for manipulating magnetic fields of RF signals in an MR system;
Figure 4A shows a composite device for manipulating a magnetic field of RF radiation from one or more RF antenna in an MR system;
Figure 4B shows a cross-section view of the composite device of Figure 4A;

Figure 4C shows a cross-section view of an alternative composite device;

Figure 5 shows a Magnetic Resonance system;

Figure 6 shows results of using the Magnetic Resonance system of Figure 5 for imaging;

Figure 7 shows a method of producing a device for manipulating magnetic field of RF signals in an MR system; and

Figure 8 shows a schematic diagram of a kit for an MR system.

DETAILED DESCRIPTION

In overview, the present disclosure relates to methods for producing a device arranged to redistribute RF fields and enhance the magnetic field of incoming RF signal into certain areas, such as areas near a patient under diagnosis in an MRI system. The resonance characteristics, e.g. resonance quality factor and resonant RF frequency, of the device affect the performance of the device in the MR system as the device interacts with other features of the system, notably, the one or more RF antenna(s) of the MR system. Hence making the device in accordance with a design based on at least one characteristic of the one or more RF antenna improves the performance of the device, e.g. by permitting higher magnetic fields to be used safely thereby improving MRI contrast or reducing scan time and increasing patient throughput.

MRI field manipulation devices

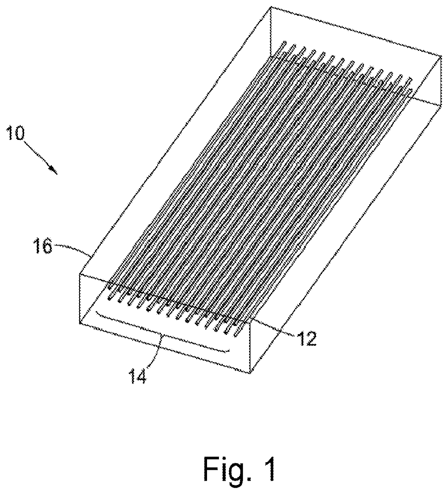

With reference to Figure 1, a device 10 suitable for manipulating the magnetic field of RF signals in an MRI system comprises a plurality of wires 12 arranged in an array 14. The wires 12 are supported by a dielectric layer 16. The wires are elongate conductive elements, having a length in a first direction much longer than the width and height dimensions. The wires are made from a non-magnetic or non-ferrous metal. The longitudinal axes of the wires 12 are substantially parallel.

The wires 12 are arranged in a two-dimensional periodic array 14, having the wires 12 evenly spaced apart in two dimensions along the height and width of the device 10. As shown in Figure 1, the array 14 comprises two rows of fourteen wires 12. The array 14 of wires 12 is embedded in the dielectric layer 16, which supports the wires 12 in the array and positions each wire 12 with respect to each other. In alternative examples, the number of wires and/or number of rows are different from as shown in Figure 1.

The array 14 and wires 12 of the array are arranged such that, when an RF signal is incident on the array 14, wires modify the RF electric and magnetic field in the vicinity of the midpoint along the length of each wire 12.

To produce the field redistribution phenomenon, the length of each wire is selected to meet the Fabry-Perot condition for the first eigenmode at the operating frequency of an MRI system. This condition is also known as half-wavelength resonance, since the length corresponds to approximately half of the wavelength in the medium of the operating frequency. For example, for 1.5 T MRI machine the operating frequency is equal to 63.8 MHz. The length of wires 12 of the device 10 can be selected using the following equation:

$$f = \frac{c}{2\,L\,\sqrt{\varepsilon}} \qquad (1)$$

where $\varepsilon$ is the relative permittivity of the environment that the conductive elements (e.g. wires) are in, L is the length of each conductive element, c is the speed of light, and f is the resonant frequency. Relative permittivity may also be referred to as the dielectric constant of a material. The permittivity of the environment of the wires is affected primarily by the permittivity of the material in which the wires are embedded, and other nearby materials may also affect this value. If the wires are embedded in more than one material, the resulting effective permittivity of the environment is calculated from the combination of the relative permittivity of each of the surrounding materials. For a frequency of 63.8 MHz in a medium with dielectric constant 81, using equation provides a wire length of 26.1cm. Note that this is less than the wavelength corresponding to the operating frequency, i.e. the frequency of the RF signal for which the device is arranged to concentrate the magnetic field. Since the elements are elongate, the width and height are therefore also subwavelength. As an alternative to using equation 1, the appropriate length for a given frequency can be determined by experimentation or simulation.

For the first Fabry-Perot mode, the largest magnetic field is localized in the middle part of the surface of the device 10 and the electric field is localized near the edges of the wires 12. The first Fabry-Perot mode is modified due to the nearfield mutual coupling between wires, but the mode structure of an array is very close to the mode structure of the single wire for the half wavelength resonance frequency. In particular, there is a maximum of the magnetic field near the centre and the maxima of the electric field are localized near the ends of wires 12.

A device as described above can be used in Magnetic Resonance (MR) systems (including MRI systems and Magnetic Resonance Spectroscopy, MRS, systems) to improve the RF signal for imaging an object. This is because an increased magnetic field in the region of the object to be imaged increases the SNR and decreasing the electric field in the region reduces the SAR. The specific embodiments disclosed herein are described primarily in context of MRI systems, but are likewise applicable to MRS systems.

Collections of subwavelength conductive elements arranged in an array to perform a particular manipulation on incoming radiation are known generally as metamaterials. The principles disclosed herein are applicable to any metamaterials used for concentrating the magnetic field of an RF signal in an MR system.

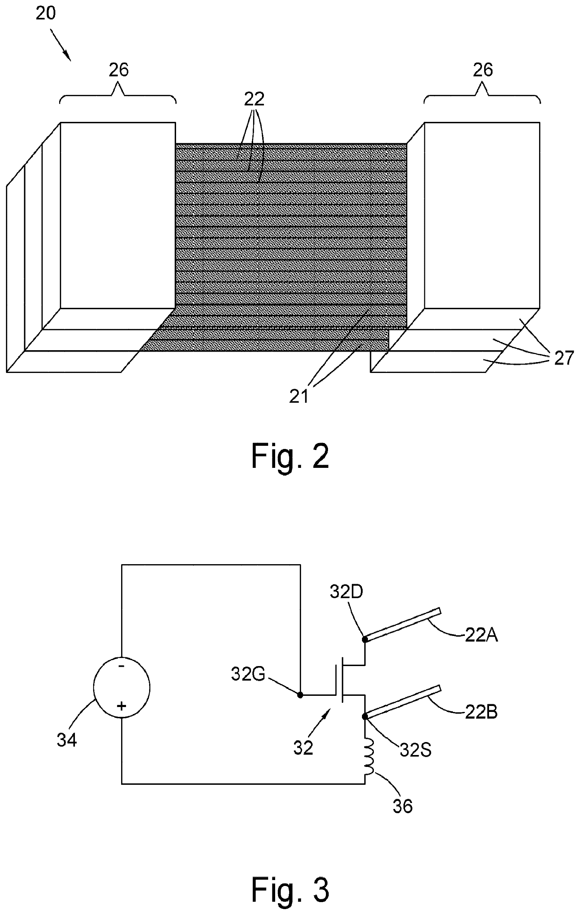

With reference to Figure 2, a device 20 suitable for manipulating the magnetic field of RF signals in an MRI system comprises a first and a second printed circuit board (PCB) 21. The first and second PCBs each have a plurality of metal strips 22. The metal strips form an array of conductive elements, wherein the array is arranged to redistribute energy between electric and magnetic fields of the RF radiation at a resonant RF frequency when receiving the RF radiation. The thickness of the metal strips 22 is less than the wavelength of RF frequency which at the array is arranged to function. The metal strips 22 can have any of the properties described above for the wires 12 as described with reference to Figure 1. In an example, there are ten metal strips 22 arranged on each of the first and second PCBs. In other examples, the number of metal strips may be different.

A dielectric material 26 is located at either end of the plurality of metal strips 22, at opposite ends of the length of each metal strip 22. The dielectric material comprises a plurality of ceramic blocks 27 placed above and below each PCB 21. Hence the dielectric material 26 substantially surrounds the ends of the metal strips 21. The PCBs 21 and dielectric material 26 can all be supported by a housing to hold the relevant components in position with respect to each other.

The metal strips 22 on the PCBs 21 have a length in accordance with the Fabry-Perot condition for one of the eigenmodes at the operating frequency of an MRI system as described above in reference to Figure 1. However, the presence of the dielectric material 26 affects what RF wavelength each of the resonances can occur at, when that RF wavelength is impinging on device. In particular, for a given frequency, the greater the dielectric permittivity of the dielectric material, or the greater coverage of the dielectric material 26 on the metal strips 22, the shorter the length of metal strips 22 that will experience resonance at the given frequency. Put another way, for a fixed length of the metal strips 22, a higher dielectric permittivity or greater coverage of dielectric material will decrease the RF frequency that experiences a particular resonance. Accordingly, the positioning of the dielectric material and the dielectric permittivity are two parameters which, individually or together, affect the resonant RF frequency of the device. Another parameter which affects the resonant RF frequency is the surface area of the conductive elements that the dielectric material covers, the greater the surface area the greater the effect of the dielectric material.

The precise effect that a particular dielectric material arrangement, e.g. positioning and/or dielectric permittivity, can be understood intuitively from equation 1 above. The resonant RF frequency is inversely proportional to the square root of the relative dielectric permittivity of the environment. However, this is based on the assumption that the dielectric material substantially encompasses the entirety of the metal strips. However, the extent of coverage of the dielectric material over the metal strips 22 will change the effective dielectric permittivity that the metal strips experience. The effective dielectric permittivity is the value for relative dielectric permittivity that would produce the RF resonant frequency according to equation 1. For example, if only the ends of the metal strips 22 are covered by the dielectric material as described with reference to Figure 1, the effective dielectric permittivity will be less than the relative dielectric permittivity of the dielectric material, since part of the surroundings of the metal strips is air, having a relative dielectric permittivity close to 1, and part is the dielectric material. The resonant RF frequency of a particular device, having a particular dielectric permittivity of dielectric material and a particular amount of coverage of the dielectric material on the metal strips 22, can be determined using calculation, simulation or by empirical measurement.

Calculation of the resonant RF frequency can be performed by modifying equation 1 above to use effective relative dielectric permittivity instead of relative dielectric permittivity, e.g. by using an average of the relative dielectric permittivity around the metal strips 22 taking into account the proportions that air and the dielectric material surround the metal strips 22. Simulation of the resonant RF frequency can be performed using electromagnetic wave simulation software, e.g. by solving the electromagnetic wave equation derived by Maxwell's equations using Finite-difference time-domain (FDTD) methods. Empirical measurement can also be performed by manufacturing a test device and detecting at which RF frequency the resonant properties occur by measuring the fields using electromagnetic probes.

The device may be an adjustable device, e.g. the device can have a variable resonant frequency by having an adjustable dielectric material. For example, the ceramic blocks 27 may be slidable to adjust their positioning on the metal strips 22, thereby changing the affect of the dielectric material 26 on the resonant RF frequency. This may be done using an adjustment mechanism, such as having the ceramic blocks 27 threaded on tracks in the device housing so that they can be slid along the length of the metal strips or removed entirely. The ceramic blocks 27, PCBs 21, or device housing may include wheels to facilitate movement of the ceramic blocks relative to the PCBs and metal strips 22 thereon. The ceramic blocks may be moveable manually by an operator, or the device may include an actuator to control the positioning of the ceramic blocks 27 in response to a user input or instructions from a processor.

The resonance of the metal strips 22 on the PCBs has a resonance quality factor, which characterises the resonance of the device. The resonance quality factor, or Q factor, is a measure of the sharpness of the resonance peak in frequency-space. In some respects, a higher quality factor indicates a greater 'strength' of resonance. One definition for the quality factor of a device as described with reference to Figure 1 or 2 is:

$$Q = \frac{f_r}{\Delta f} \qquad (2)$$

wherein Q is the resonance quality factor, $f_r$ is the resonant RF frequency value and $\Delta f$ is the resonance width. The resonance width may be defined as the full width at half maximum (FWHM) of the resonance peak in frequency space.

The resonance quality factor of the device, in general, depends on one or more of the following parameters of the device. The resonant RF frequency, as described above, depends on the length of the metal strips 22, the dielectric permittivity of the dielectric material 26 and the positioning of the dielectric material 26 (relative to the plurality of conductive elements). The resonance width depends on a loss tangent of the dielectric material 26. For example, a dielectric material having a high loss tangent will produce a higher resonance width and accordingly a lower resonance quality factor. As an example the resonance quality factor of the devices may be between 10 and 500, optionally between 50 and 150. The resonance quality factor of a device may be determined using simulation techniques, e.g. Finite-difference time-domain (FDTD) methods, or measurements as described above.

The loss tangent of the dielectric material 26 can be defined as:

$$\tan \delta_e = \frac{\varepsilon''}{\varepsilon'} \qquad (3)$$

where $\tan \delta_e$ is the electric loss tangent, $\varepsilon''$ is the imaginary component of the complex dielectric permittivity and $\varepsilon'$ is the real component of the dielectric permittivity (which is also the relative dielectric permittivity multiplied by the permittivity of free space $\varepsilon_0$). The loss tangent of the dielectric material 26 may be a fundamental property of the material or can be designed by adding dopants to the dielectric material 26. Typically, the loss tangent is between 0.1 and 0.001 (unitless). A loss tangent of 0.1 would indicate a relatively lossy material, whereas 0.001 would be relatively low loss.

Another property which can affect the resonant RF frequency and/or the resonance quality factor is one or more electronic components connected between two respective portions of the conductive elements. For example, the electronic components may be connected between two conducive elements, between different portions of a single conductive elements, or to one or more external conductive element not part of the array of conductive elements.

As an example, with reference to Figure 3, a device with controllable resonance quality factor has a potentiometer 34 arranged to control a signal which determines the bias voltage of a transistor 32 connected between a pair of metal strips 22A and 22B. One end of the potentiometer 34 is connected to the transistor gate 32G and the other end is connected to the transistor source 32S via an inductor 36. The transistor drain 32D and transistor source 32S are connected to the pair of metal strips. The signal from the potentiometer determines the capacitance of the transistor between conductive elements and the resonant RF frequency of the device. The change in capacitance in the transistor also affects the loss in the metal strips 22. An increased loss in the metal strips 22 produces a lower resonance quality factor of the device.

A DC power is input to the potentiometer 34 so that the controlled resistance of the potentiometer 34 controls the gate voltage of transistors 32. Hence the potentiometer 34 supplied with a DC power input acts as a variable DC voltage supplier. Alternative variable DC voltage suppliers may be used instead of the potentiometer 34. When the transistor is reverse biased, varying the gate voltage varies the bias voltage and therefore varies the capacitance between the drain and source of the transistor. This in turn varies the impedance, i.e. a conduction state, of the transistor. Hence the potentiometer 34 controls the conduction state of the transistors. Accordingly, controlling the resistance setting of the potentiometer 34 controls the capacitance between the metal strips 22 of the device, and therefore controls the resonant frequency and resonance quality factor of the device. Consequently, varying the resistance setting of the potentiometer 34 will tune or de-tune the frequency at which the device manipulates the magnetic field of RF signals in an MRI system, and will control the quality factor of the resonance. The potentiometer 34 can be controlled using control signals from other components in the MRI system, either wirelessly or via electronic connection.

Instead of the potentiometer and transistor arrangement, other electronic devices and controllers for controlling the electronic device may be arranged between the conductive elements in order to control the resonance properties of the device 20.

For example, instead of a transistor 32, a potentiometer can be connected between conductive elements, or portions thereof. The potentiometer produces a variable and controllable electrical resistance between the two conductive elements or portions thereof. An increase in resistance between conductive elements of the device will increase the resonance quality factor of the device (i.e. reduce the loss tangent), and a decrease in resistance will decrease the resonance quality factor of the device (i.e. increase the loss tangent).

As another example, the resonant frequency of device can be varied by controlling electronic components electrically connected between the metal strips and respective metal strip extensions located at the ends of the metal strips. By controlling the conduction properties of the electronic components, the effective length of the metal strips will change, e.g. to the combined length of the metal strip and corresponding metal strip extension, and therefore change the resonant frequency of the device, as understood from equation 1.

The devices for manipulating a magnetic field of RF radiation from one or more RF antenna described above with reference to Figures 1 to 3 may, in alternative arrangements, have the following variations.

The devices may have only a single PCB 21 with metal strips 22 thereon, which may simplify manufacture. The thickness of the one or more PCB may be between 0.127mm and 1mm, optionally 0.254mm. The PCB may be a FR4-PCB or a Rogers 3010™ PCB. The metal strips 22 printed on the PCB may be copper, or another conductive non-magnetic metal. The metal strips are preferably thicker than the skin depth of the selected metal at the frequency that the device is designed for, e.g. greater than 10$\mu$m for copper. In an example, each PCB has 10 metal strips each 35$\mu$m thick, the metal strips are 2mm wide and spaced apart on the PCB at intervals of 10mm, i.e. 10mm between each pair of adjacent metal strips. Alternatively, the spacing of the metal strips may vary across the PCB and may be non-periodic.

The dielectric material 26 may be made from ceramic blocks 27 made from $BaTiO_3$ or $CaTiO_3$, or a combination thereof. Instead of multiple ceramic blocks 27, the dielectric material 26 may be formed as a single unit into which the one or more PCB can be inserted. The dielectric material 26 may be powders of $BaTiO_3$ or $CaTiO_3$, or other dielectric material, and either formed into blocks or held inside dielectric material containers. The dielectric material 26 may be formed of any dielectric material which affects the resonance properties of the device. The relative dielectric permittivity of the dielectric material may be greater than 50, or greater than 100. In an example, the ceramic blocks 27 each have a thickness of 10mm, a width of 100mm a length of 180mm and a relative dielectric permittivity of 108.

The dielectric material 26 may be located at only one end of the metal strips 22. Alternatively, the dielectric material 26 may be positioned along the metal strips 22 to substantially cover the entire length of the metal strips, i.e. not only be located at the ends of the metal strips 22. The dielectric material 26 may comprise two or more portions each containing material having a respective relative dielectric permittivity. Instead of oblong blocks, the dielectric material 26 may have any shape, e.g. spheroid, cylindrical, etc.

The design of the device for manipulating a magnetic field of RF radiation from one or more RF antenna in an MR system, in general, includes any parameter which affects the resonance properties of a device made according to the design, in particular, the resonant RF frequency of the device and the resonant RF quality factor of the device. The design of the device may include a subset of all such possible parameters wherein the subset substantially determines the resonant RF frequency of the device and/or the resonant RF quality factor of the device. For example, the design of the device may include all of the variable parameters of the device, or just one parameter. If the design is to make a device which has predetermined dimensions of the PCBs 21 and metal strips 22, these are treated as fixed parameters, then the design of the device may not include these parameters.

The design of the device comprises any combination of one or more of: a positioning of the dielectric material 26; the dielectric permittivity of the dielectric material 26; the loss tangent of the dielectric material 26; and one or more electronic components connected between two respective portions of the conductive elements (e.g. the details of if and where one or more transistor 32 should be placed between metal strips 22).

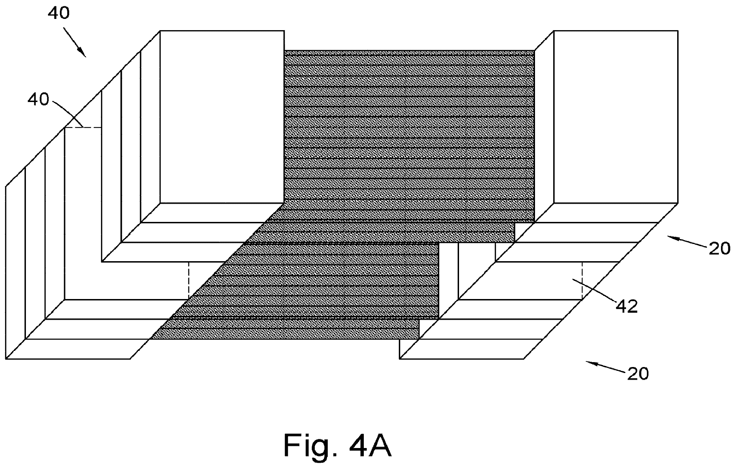

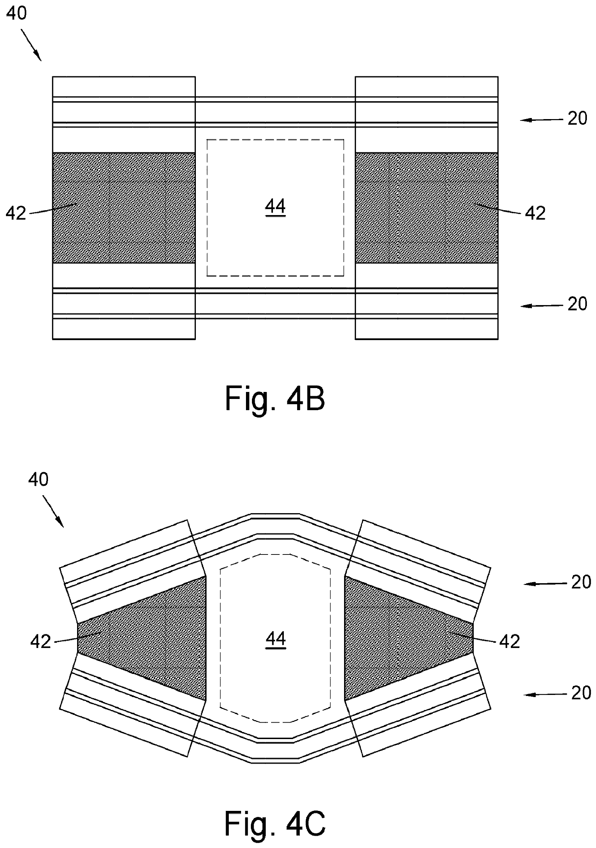

With reference to Figure 4A, a composite device 40 for manipulating a magnetic field of RF radiation from one or more RF antenna in an MR system comprises a first component device 20 and a second component device 20, each component device being as described above with reference to Figure 3. In particular, each component device 20 of the composite device 40 comprises PCBs 21 having metal strips 22 and a dielectric material 26. The two component devices 20 are separated by two spacers 42 which support component devices 20. With reference to Figures 4B and 4C, between the two component devices there is an interior region 44 for receiving an object to be imaged in an MR system. The two component devices 20 may have the same dimension and resonance properties. The spacers 42 may be made from a dielectric material to match the dielectric material of one or both of the component devices 20.

With reference to Figure 4B, the spacers 42 are be oblong and placed between the ceramic blocks 27 of the component devices 20, creating the interior region 44 near a middle portion of the length of the metal strips 22 of each component device 20. This improves the uniformity of the magnetic field manipulation in the interior region 44, thereby providing improved image uniformity when used in an MRI system.

Alternatively, with reference to Figure 4C, the spacers 42 are trapezoid and the component devices 20 curve near a middle portion of the metal strips 22 of each component device 20. The curves of the component devices curve outwardly from the interior region 44, so that the distance between the respective middle portions of the first and second component devices is greater than the example described above with reference to Figure 4B. This may provide a more suitable interior region 44 for receiving spheroid or substantially cylindrical objects to be imaged, e.g. a patient's head or limbs. Having two component devices 20 also improves the uniformity of the magnetic field manipulation in the interior region 44, thereby providing improved image uniformity when used in an MRI system.

### MRI system

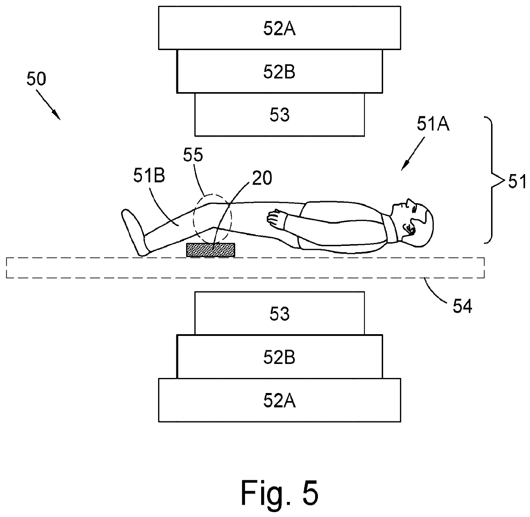

An MRI system comprising a device 20 as described above will now be described with reference to Figure 5.

An MRI system 50 comprises an imaging region 51 arranged to receive an object to be imaged, e.g. a human body 51A or human limb 51B. A first coil 52A produces a static magnetic field in the imaging region 51 and, in operation, a gradient coil 52B produces a gradient to static magnetic field in the imaging region. Together, the first coil 52A and gradient coil 52B are a magnetic field generator 52. The system further comprises an RF antenna 53 for irradiating the object with an RF signal. The RF antenna 53 is arranged to transmit RF signals as a pulse and then have a delay between pulses during which the return RF signal is received. The RF antenna 53 may be a RF transmit coil built into a housing supporting the first coil and gradient coil, or may be a separate RF antenna placed at a location for irradiating the object in the imaging region 51. A table 54 is located in the imaging region 51 to support the object to be imaged. The device 20 for concentrating the magnetic field of RF signals in the MRI system 50 as described above is located in the imaging region 51 in proximity of the object, or a particular target region 55 of the object to be imaged. The device is arranged to concentrate the magnetic field of RF signals in the object to imaged. The device is arranged between the RF antenna 53 and object so, if tuned to the RF signal frequency, the device 20 concentrates the magnetic field of the RF signal from the RF antenna 53 to the object in the target region 55, thereby improving the SNR. As described above, this is by redistributing the energy between electric and magnetic fields of the RF signal, increasing the magnetic field in the target region 55 and reducing the electric field in the target region 55 which reduces the SAR.

The RF antenna 53 may also function as an RF receiver, with the return signal from the object being recorded to image the object. Alternatively, the table 54 may comprise a dedicated coil 56 (not shown) which functions as an RF receiver as it receives the return signal in order to image the object. In either arrangement, when the device is positioned between the object and the RF receiver (tuned to the RF signal), the device 20 will also concentrate the magnetic field of the return signal as it passes from the object to the RF receiver. The system may comprise a plurality of RF antennas 53 to transmit and/or receive the RF signals.

The device 20 may be fixed on, or embedded in, the table 54 or may be a mat which is laid on the table 54 prior to introducing the object to be imaged into the imaging region. Alternatively, the device may be placed on the object, e.g. in an item of clothing worn by a patient.

The device 20 is arranged to resonate with a resonance quality factor and/or the resonant RF frequency based on the at least one characteristic of the one or more RF antenna 53. Characteristics of the one or more RF antenna may include one or more of: an RF frequency that the one or more RF antenna is arranged to transmit; a resonance quality factor of the one or more RF antenna;

and an object to be imaged by the one or more RF antenna, a dielectric constant of the object to be imaged, or a combination thereof. For example, the characteristics of the RF antenna 53 may be that it is arranged to provide RF radiation at approximately 63.8 MHz, has a resonance quality factor of 100, and is arranged to image a patient's head. These are characteristics which can have an effect on the resonance performance of the device 20, e.g. the resonant RF frequency or the resonance quality factor, as explained below.

In order to produce the beneficial magnetic field manipulation effect, the device 20 must resonate at and around the frequency used by the RF antenna 53 to redistribute energy between the electric and magnetic fields. The RF antenna frequency influences the design of the device 20 and, in particular, a target resonant RF frequency of the device. Likewise, the type of object the RF antenna is arranged to image will affect the resonant RF frequency of the device, since different objects have dielectric properties which can shift the resonance properties of the device when placed near, e.g. during imaging. For example, an object with a higher dielectric constant will reduce the resonant RF frequency of the device 20 more than a lower dielectric constant object. Hence the design of the dielectric material used for the device may be based at least in part on the object which the RF antenna is arranged to image.

As another example, if the resonance quality factor is above a threshold quality factor for the RF antenna 53, e.g. if the resonance quality factor of the device is greater than the resonance quality factor of the RF antenna 53, the resonance effect will produce particularly high fields in the imaging region 51 which will disrupt the performance of the first coil 52A and/or the gradient coil 52B of the magnetic field generator 52. This can be caused by the control systems of the magnetic field generator 52 of the MRI system 50 being programmed to maintain a uniform magnetic field in the imaging region 51. The presence of a resonating device 20 having a high resonance quality factor therefore disrupts the uniformity of the magnetic field, which may either prompt the control system to prevent the MRI system 50 or magnetic field generator 52 from obtaining an image, or create an unexpected magnetic field that renders the MRI system unusable for imaging. Additionally or alternatively, a high resonance quality factor of the device 20 may detune the RF antenna, first coil 52A, or gradient coil 52B. The design of the device may therefore produce a resonance quality factor of the device 20 in order to not disrupt the magnetic field of the MRI system by not detuning the one or more RF antenna 53 or magnetic field generator 52. This improves the reliability of the device 20 and system 50.

In operation, the MRI system 50 may be used to image an object using the RF antenna 53 and device 20 as follows. The device 20 is positioned in or adjacent to the imaging region 51 so to manipulate the magnetic field near the object to be imaged. As an example, with reference to figure 5, the device 20 is placed on the table 54 outside the imaging region 51 of the MRI system at a location where a knee of the human body 51A (i.e. the patient) to be imaged will be located. The patient then is positioned on the table 54 with the knee to be imaged over the device 20 and the table 54, along with the patient and device 20, is positioned into the imaging region 51 prior to commencing the imaging process. Other examples of body parts the MRI system can be used to image include a wrist, a spine, etc. or indeed the MRI system can image an entire body. For the MRI process to begin, a static magnetic field is produced in the imaging region, optionally having a gradient field according to known MRI techniques.

The MRI system 50 irradiates the device 20 and object with RF radiation as an RF signal from the RF antenna 53 and receives a return RF signal from the object to image the object. The irradiating may comprise transmitting the RF signal as an RF pulse. The RF pulse travels to a target region 55 of the object to be imaged via the device 20. If the device 20 is tuned to the frequency of the RF signal, the device concentrates the RF signal in the target region 55 by increasing the magnetic field and reducing the electric field. After impinging on the target region 55, the RF signal is emitted from the target region 55 as a return RF signal. The return RF signal passes through the device 20 again on return to the RF antenna 53 for detection and imaging of the target region 55. The device may also concentrate the return RF signal by increasing the magnetic field and reducing the electric field from the target region 55.

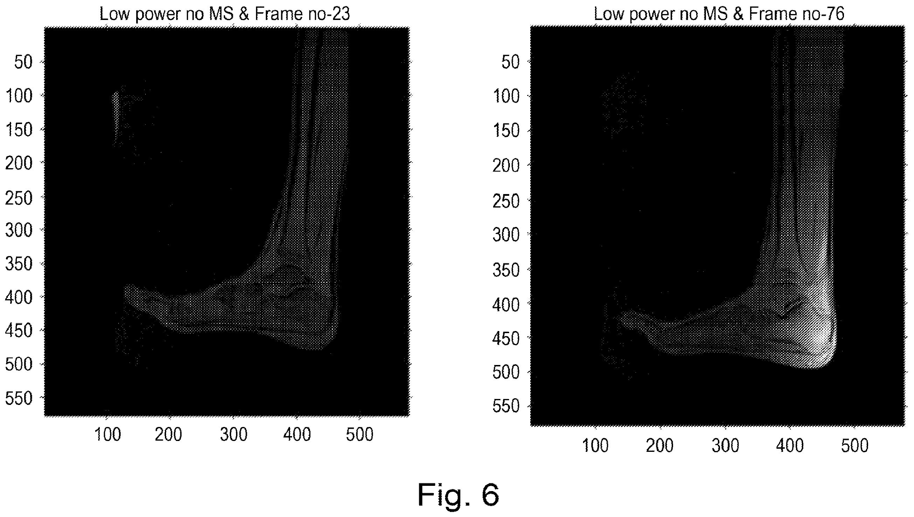

Figure 6 shows a comparison of MRI images produced in an MRI system with and without a device 20 as described above. The left image shows an MRI image produced without a device 20 for manipulating the magnetic field of RF radiation and the right image shows an MRI image produced with the device 20. The image produced using the device 20 has improved contrast in the target region 55 of the object, in this case, part of the patient's foot and ankle. This is due to the increased magnetic field in the target region of the object.

Methods of producing the device

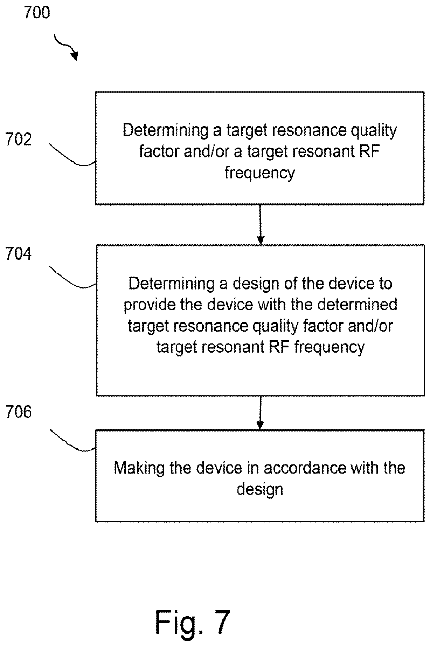

With reference to Figure 7, a method 700 of producing a device for manipulating a magnetic field of RF radiation from one or more RF antenna in an MR system comprises: determining 702 a target resonance quality factor and/or a target resonant RF frequency of the device; determining 704 a design of the device to provide the device with the determined target resonance quality factor and/or target resonant RF frequency of the device; and making 706 the device in accordance with the design. The device to be produced can be any device as described above for use in an MR system as described above.

The determining 702 a target resonance quality factor and/or a target resonant RF frequency of the device is based on at least one characteristic of the one or more RF antenna of the MR system, such as an MRI system as described above with reference to Figure 5.

As an example, a target resonance quality factor of the device is based on a resonance quality factor of an RF antenna of an MRI system, i.e. the resonance quality factor of the RF antenna is the characteristic, as explained below.

A user input is received at a processor, the user input defining a resonance quality factor of a RF antenna of the MR system which is a characteristic of the RF antenna. The user input is received at a user interface associated with the processor. The user input may provide the characterisitc of the RF antenna by including a numerical value, or a numerical range, of the resonance quality factor of the RF antenna, e.g. specifies a resonance quality factor of 90, or between 50 and 100, or between 80 and 100. The processor then determines the target resonance quality factor of the device to be produced based on the value of the the resonance quality factor of the RF antenna provided by the user input. For example, this is done by using a target criterion such as the target resonance quality factor of the device is determined to be less than the resonance quality factor of the RF antenna. Alternative criteria include: that the target resonance quality factor of the device is determined to be below a certain percentage of the resonance quality factor of the RF antenna; or that the target resonance quality factor of the device is determined to be approximately a certain percentage of the resonance quality factor of the RF antenna, e.g. approximately 80% of the value of the resonance quality factor of the RF antenna.

The criterion or criteria to determine the target resonance quality factor of the device may be such that that the device does not detune the one or more RF antenna when used in the MR system. This is to avoid the phenomenon, as described above with reference to Figure 5, of the device 20 for manipulating a magnetic field of RF radiation from disrupting the performance of the RF antenna 53 or the magnetic field generator 52 of the MRI system 50. Accordingly, determining the target resonance quality factor of the device based on the characteristic of the RF antenna, namely a resonance quality factor, improves the reliability of the device to be produced.

Other characteristics of the RF antenna that the target resonance quality factor of the device is based on include an RF frequency that the RF antenna is arranged to transmit; the dimensions of the RF anteanna; the power source used to power the RF antenna; an object to be imaged by the RF antenna; a loss tangent of the object to be imaged by the RF antenna; and a dielectric contstant of the obejct to be imaged by the RF antenna; or any other characterisitic which can affect the interactions between the device 20 to be produced and the RF antenna 53 of the MRI system 50.

As another example of the determining 702 in Figure 7, the target resonant RF frequency of the device is determined based on an RF frequency that the RF antenna 53 is arranged to transmit. i.e. the resonant RF frequency of the RF antenna is the characteristic, as explained below.

The resonant RF frequency of the RF antenna is received at the processor in a user input, similar to as described in the previous example, except that the user input comprises a numerical value or range for the resonant RF frequency that the RF antenna is arranged to transmit. For example, the value or range may be that the RF antenna 53 is arranged to transmit at 63.8 MHz, or between 60 MHz and 65 MHz. The processor stores the received numerical value or numerical range from the user input as the target resonant RF frequency. The processor then determines the target resonant RF frequency the device to be produced based on the value or range of the RF frequency of the RF antenna provided by the user input. For example, the processor may store the received numerical value or numerical range from the user input as the target resonant RF frequency. This is so that the device 20 to be produced will provide the effect of manipulating the magnetic field of the RF radiation from the RF antenna, i.e. by resonating at the frequency that the RF antenna transmits. The target resonant RF frequency may be the range between 95% and 105% of the RF frequency of the RF antenna. Alternatively, the processor may apply a criteria or calculation to determine the target resonant RF frequency for the device. For example, the target resonant RF frequency may be 95% of the value of the RF frequency of the RF antenna, or 105% of the value of the RF frequency of the RF antenna. This may be done to control the resonance strength of the device 20.

Other characteristics of the RF antenna that the target resonant RF frequency of the device is based on include: a resonance quality factor of the the RF antenna; the dimensions of the RF antenna; the power source used to power the RF antenna; an object to be imaged by the RF antenna; a loss tangent of the object to be imaged by the RF antenna; a dielectric contstant of the object to be imaged by the RF antenna; and/or any other characterisitic which can affect the interactions between the device 20 to be produced and the RF antenna 53 of the MRI system 50. For example, the a resonance quality factor of the the RF antenna, which is related to the frequency width of the RF antenna frequency peak, may influence how close the target resonant RF frequency should be to the RF frequency of the RF antenna. The type of object that the RF antenna is arranged to image, or the loss tangeant and/or dielectric constant of the object, will affect the level of detuning of the device resonant RF frequency when the object is placed in proximity with the device 20 during imaging. Hence the target resonant RF frequency can be determined to based on these parameters of the object to be imaged by the RF antenna in order to provide the desirable resonance characteristics of the device 20 in use, thereby improving the performance of the device. For example, if the RF antenna 53 is for imaging a high dielectric constant object, the target resonant RF frequency can be determined to be higher than the RF frequency of the RF antenna 53 in accordance with equation 1. Therefore, when used with the object, the decrease in resonant RF frequency of the device caused by the object will re-tune the device 20 to the RF frequency of the RF antenna 53.

In either of the above examples of determining 702, the determined target resonance quality factor and/or the determined target resonant RF frequency may be a range of values.

As part of the determining 702, the processor may receive an RF antenna identifier associated with the RF antenna 53 the device is to be used with. The RF antenna identifier is associated with further characteristics of the RF antenna stored in a memory, e.g. as part of a look-up table. The determining 702 may comprise the processor retrieving characteristics of the RF antenna from the memory using the RF antenna identifier and using the retrieved characterisitcs to base the determining of target resonance quality factor on.

As part of the determining 702, the processor may measure the characteristics of the RF antenna 53, e.g. using an electromagentic field sensor arranged to receive RF radiation from the RF antenna. The measuring may also comprising controlling an input to the RF antenna to measure performance over a range of frequencies.

As part of the determining 702, the processor may include calculating the target resonance quality factor and/or target resonant RF frequency using an equation based on the at least one characteristic of the one or more RF antenna.

As an alternative to using predetermined criteria for determining 702 the target resonance quality factor and/or target resonant RF frequency, simulation can be used. For example, the characteristics of the RF antenna 53 are input into a simulation, e.g. a Finite-difference time-domain (FDTD) simulation, along with trial values for the target resonance quality factor and/or target resonant RF frequency. The RF antenna 53 and a device 20 having the trial values are simulated and the results of the simulation show the performance of the device, which can be assessed to determine whether or not the trial values should become the target value for the device to be produced. The assessment may be based on any particular desired outcome but, as an example, this may be that RF antenna 53 produces resonance in the device 20 and the device 20 does not detune any of the other components. The magnetic field generator 52 and object to be imaged may also be included in the simulation.

In some examples, the system in which the device 20 is arranged to be used has a plurality of RF antennas. In these examples the determining 702 a target resonance quality factor and/or target resonant RF frequency of the device can be based on one of the plurality of RF antennas, or on two or more of the RF antennas. For example, the target resonance quality factor may be determined to be less than the lowest value of each of the resonance quality factors of the RF antennas, or to be less than an average value of the resonance quality factors of the RF antennas. The determining 702 a target resonant RF frequency of the device 20 can be based on an average of the resonant RF frequencies of the RF antennas, or based on an average dielectric constant of the one or more objects to be imaged by the respective RF antennas, or any other criteria for optimising performance of the system for which the device 20 is arranged to be used with.

With reference to Figure 7, the determining 704 the design of the device is to provide the device with the determined target resonance quality factor and/or target resonant RF frequency of the device, as determined using any of the approaches described above.

As discussed above, with reference to Figure 2 when describing devices for manipulating a magnetic field, the design of the device 20, in general, includes one or more parameters which affect the resonance quality factor and/or resonant RF frequency of a device made according to the design. For example, the design may define one or more of: the dielectric permittivity of the dielectric material; the loss tangent of the dielectric material 26; one or more electronic components connected between two respective portions of the conductive elements 22; a length of the conductive elements 22; a proportion of the length of the conductive elements 22 along which the dielectric material 26 is positioned; the composition of the dielectric material 26, or any parameter which affects the resonant properties of the device 20. Optionally, the design may also include further properties of the device 20 to be made, such as: the materials of the conductive elements 22, the dimensions of the device, the presence and size of any spacers 42 to be used, etc. Any features of the device to be made not specified by the design may be given a default value, or may be specified using additional user inputs.

As an example, the determining 704 the design in order to provide the determined 702 target resonance quality factor includes determining the loss tangent of the dielectric material 26 of the device 20 to be produced. This may be done by the processor retrieving from a memory a value of loss tangent of the dielectric material to provide the target resonance quality factor, or if the target resonance quality factor is a range, a value within the range. Alternatively, the loss tangent may be calculated using an equation relating loss tangent to resonance quality factor of the device, e.g. an equation derived empirically from measurements of devices having different loss tangents. Alternatively, the processor may perform a simulation, e.g. a FDTD simulation, with a trial loss tangent value and default values for any other relevant parameters of the device 20 and input RF radiation. The resonance quality factor of the simulated device can be calculated using equation 2. If the resonance quality factor the simulated device provides the determined 702 target resonance quality factor, then the design of the device 20 is determined to include the trial loss tangent. If the resonance quality factor of the simulated device does not provide the determined 702 target resonance quality then a different trial loss tangent is used in a further simulation until a value is found which provides the target resonance quality factor, which will then be used for the design of the device 20. Optionally, determining the design may also include selecting a material to use for the dielectric material 26 which has the determined loss tangent of the design. Another alternative is to make a trial device having the trial loss tangent and measuring the resonance quality factor of the trial device using input radiation and sensors to detect the resonance properties of the trial device.

If the design of the device includes multiple parameters affecting the resonance quality factor of the device 20, then these parameters may all be determined, either one-by-one or collectively, using the above methods. For example, all of the parameters can be simulated until a design to produce the target resonance quality factor is determined. Alternatively, the processor may retrieve sets of values from the memory, which produce the target resonance quality factor when used in the design.

As another example, the determining 704 the design in order to provide the determined 702 target resonant RF frequency includes determining the positioning of the dielectric material 26, e.g. the proportion of the length of the conductive elements 22 along which the dielectric material 26 is located. As described above for determining a design to provide a target resonance quality factor, determining the design to provide a target resonant RF frequency may be done by calculation, retrieval from memory, simulation or measurement using a similar process to as previously described. For example, equation 1 can be used to calculate the proportion of the length of the conductive elements 22 along which the dielectric material. In particular, the relative dielectric permittivity $\varepsilon$ of the environment that the conductive elements are in can be approximated by:

$$\varepsilon \approx P\,\varepsilon_d + (1-P)\varepsilon_a \qquad (4)$$

where P is the proportion of the length of the conductive elements 22 along which the dielectric material 26 is located (0 < P < 1), $\varepsilon_d$ is the relative dielectric permittivity of the dielectric material 26 and $\varepsilon_a$ is the dielectric material of air (or whatever substance surrounds the portion of the conductive elements where the dielectric material is not located). For example, using equations 1 and 4, to provide a device with resonant RF frequency of 62 MHz, with conductive elements having length of 50cm, dielectric material relative dielectric permittivity of 75, the required proportion of the length of the conductive elements 22 along which the dielectric material 26 is located is approximately 0.3, or 30%, e.g. 15% of the length at each end of the conductive elements. This is because the effective relative dielectric permittivity is 23.2 = (0.3*75) + (0.7*1) using equation 4 and the resonant RF frequency using equation 1 is 62.3 MHz = c / (2*0.5*V23.2). Alternatively or additionally, the dielectric permittivity of the dielectric material and/or the length of the conductive elements can be determined for the design using the equations 1 and/or 4.

If the design of the device includes multiple parameters affecting the resonant RF frequency of the device 20, then these parameters may all be determined, either one-by-one or collectively, using the above methods. For example, all of the parameters can be simulated until a design to produce the target resonant RF frequency is determined. Alternatively, the processor may retrieve sets of values from the memory, which produce the target resonant RF frequency when used in the design. If the design of device is to produce both a target resonance quality factor and a target resonant RF frequency, then further parameters affecting either or both the resonant RF frequency and resonance quality factor are calculated or simulated together to provide the target resonance properties.

With reference to Figure 7, the determining 706 the making the device in accordance with the design may include assembling the conductive elements 22, dielectric material 26 into a device 20 as described with reference to Figures 1 to 4.

As an example, the making 706 may include fabricating one or more PCBs 21 having metal strips 22 of either a predetermined length or, if specified in the design, with a length according to the design of device. Alternatively, the PCBs 21 may have been fabricated prior to the making 706 of the device. The PCBs can be made using conventional PCB fabrication techniques.

If the design of the device includes one or more electronic components connected between two respective portions of the conductive elements, e.g. metal strips 22, these may be built in to the PCBs during the fabrication process, or connected between the relevant conductive elements according to the design in a second process after the PCB has been fabricated. Either a controller of the electronic components, or an electrical input port to receive a control signal for the electronic components, is also included in the device. A power source, e.g. a battery, or a power connection, e.g. a power port, to power the controller and/or electronic components may also be included. For example, a potentiometer and DC power source as described with reference to Figure 3 may be included in the device.

The PCBs 21 are stacked with the dielectric material 26 positioned above and below each PCB 21, located at end portions of the lengths of the metal strips 22. Ceramic blocks 27 may be used for the dielectric material 26, either prefabricated or by selecting the material for the ceramic block, e.g. $BaTiO_3$ or $CaTiO_3$, and forming the material into the ceramic blocks 27. If the device to be produced is a composite device 40 as described above with reference to Figures 4A to 4C, the making 706 comprises positioning the two or more component devices 20 on either side of the one or more spacers 42.

The stack of PCBs 21 and ceramic blocks 27, and optionally spacers between component devices 20, may be held together using adhesives or mechanical fasteners such as screws or bolts. Alternatively, the items of the stack are positioned in a housing to hold the items in position with respect to each other. This may be done by the housing having a plurality of slots in which the items can be slid into.

If the device is arranged to have moving parts, such as a slidable dielectric material 26, these parts are attached to the rest of the device using a sliding mechanism. Hence the device is an adjustable device. In an example, the ceramic blocks 27 are coupled to the housing using wheels and axles attached to each ceramic block 27 arranged to engage with slots or runners in the housing. The ceramic blocks may include a handle, connected to one or more ceramic block either individually or collectively, so that a user can manually change the positioning of the ceramic blocks 27 along the slots or runners. Alternatively, an actuator is coupled to one or more of the ceramic blocks 27 to control the positioning of the ceramic blocks.

In an example of a method 700 of producing a device 20 for manipulating a magnetic field of RF radiation from one or more RF antenna 53 in an MR system 50, an adjustable device is already assembled prior to the determining of a target resonance quality factor and/or target resonant RF frequency of the device. The making 706 of the device 20 in accordance of the design comprises adjusting one or more parameters of the adjustable device in order to provide the device with the determined target resonance quality factor and/or determined target resonant RF frequency of the device 20.

For example, the method 700 may be performed during imaging of an object by an MRI system 50. The determining 702 of a target resonant RF frequency of the device may be based on measurement of the RF frequency transmitted by the RF antenna 53, or based on the dielectric properties of the object 51 into which the RF antenna 53 is transmitting (or any of the other examples provided above for determining 702). This may be performed in accordance with any of the methods previously described, or may include determining if the adjustable device resonant RF frequency is too low or too high compared to the RF frequency of the RF antenna. Hence the target resonant RF frequency may be to adjust the device to make the device either increase or decrease its resonant RF frequency.

A processor, either part of the device 20 or otherwise arranged to control the device 20, determines 704 a design of the device in order to provide the determined target resonant RF frequency of the device. For example, to produce a device 20 with a higher resonant RF frequency, the design has the dielectric material positioned along a smaller proportion of the conductive elements than the starting proportion (in accordance with equations 1 and 4). In other words, to make 706 the device 20 in accordance with the determined 704 design, the adjustable device should be adjusted to slide the dielectric material 26 away from the conductive elements so less of each conductive element is covered. On the other hand, to produce a device 20 with a lower resonant RF frequency of the device, the adjustable device should be adjusted to slide the dielectric material to cover a greater proportion of the conductive elements.

Alternatively, if the proportion of the conductive elements covered by the dielectric component does not change, e.g. in terms of Figure 2 because the ceramic blocks 27 have been slid so that their entire width covers the metal strips 22, then the location of dielectric material along the length of the conductive elements also has an effect of the resonant RF frequency. In an example, the dielectric material comprises two blocks at either end of the conductive elements. As the blocks are moved in from the ends of the conductive element, the proportion of the conductive elements that they cover increases and the RF resonant frequency decreases, as explained above. Once the entire width of the dielectric material blocks covers the conductive elements, moving the blocks further towards the midpoint of the conductive elements no longer increases the proportion of the conductive elements covered and the effective permittivity of the surroundings remains substantially constant. However, as the blocks continue to move closer to the midpoint of the conductive elements, this has a different effect of decreasing the effective length of the conductive elements, thereby increasing the resonant RF frequency. The particular effect of the positioning of the dielectric material on the resonant RF frequency can be determined empirically or by simulation, as explained above.

To make 706 the device in accordance with the design, the processor sends instructions to the actuator to adjust the proportion of the conductive elements along which the dielectric material 26 is located in accordance with the determined design. Alternatively, the processor may provide an output at a user interface indicating the adjustment of the dielectric material required for a user to manually adjust the proportion of the conductive elements along which the dielectric material 26 is located in accordance with the determined design. The user then adjusts the proportion of the conductive elements along which the dielectric material 26 is located using the handle and sliding mechanism of the adjustable device.

The method 700 of producing the device 20 may use an adjustable device having adjustable parameters other than proportion of the conductive elements along which the dielectric material is located. For example, the length of conductive elements may be adjustable by providing extensions arranged next to each conductive element with a respective switch or electronic component to connect or disconnect each extension from the respective conductive element, to adjust the effective length of the conductive elements. Alternatively, electronic components connected between two respective portions of the conductive elements, as described with reference to Figure 3, can be controlled to adjust the loss of the device and thereby changing the resonance quality factor of the device. In any of the above examples, the making 706 of the device 20 in accordance with the deign includes adjusting a parameter of the device in order to meet the design, to produce the target resonance properties.

One or more of the features of the method 700 may be computer-implemented, e.g. performed by a processor. For example, the determining 702 a target resonance quality factor and/or a target resonant RF frequency of the device based on at least one characteristic of the one or more RF antenna, and/or the determining 704 a design of the device to provide the device with the determined target resonance quality factor and/or the target resonant RF frequency of the device may be computer-implemented by a processor. The making 706 the device in accordance with the design may include the processor instructing a making apparatus to make the device in accordance with the determined design.

The methods described above may be implemented by a computer program product. The computer program product may include computer code arranged to instruct a computer to perform part of the functions of one or more of the methods described above. The computer program and/or the code for performing such methods may be provided to an apparatus, such as a computer, on a computer readable medium or computer program product. The computer readable medium may be transitory or non-transitory. The computer readable medium could be, for example, an electronic, magnetic, optical, electromagnetic, infrared, or semiconductor system, or a propagation medium for data transmission, for example for downloading the code over the internet. Alternatively, the computer readable medium could take the form of a physical computer readable medium such as semiconductor or solid-state memory, magnetic tape, a removable computer diskette, a random access memory (RAM), a read-only memory (ROM), a rigid magnetic disc, and an optical disk, such as a CD-ROM, CD-R/W or DVD.

A computer apparatus such as a computer may be configured in accordance with such code to perform one or more processes in accordance with the methods discussed herein, in combination with an apparatus for making 706 a device 20 in accordance with the design. Such a computer apparatus may take the form of a data processing system. Such a data processing system may be a distributed system. For example, such a data processing system may be distributed across a network.

In an implementation, the modules, components and other features described herein can be implemented as discrete components or integrated in the functionality of hardware components such as ASICS, FPGAs, DSPs or similar devices.

A "hardware component" is a tangible (e.g.,

non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Kit

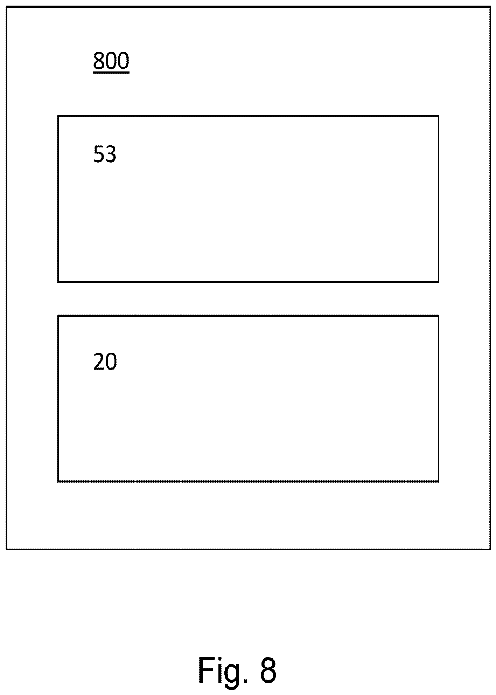

With reference to Figure 8, a kit 800 comprises at least one RF antenna 53 and a device 20 for manipulating a magnetic field of RF signals in an MR system. The device 20 is arranged to resonate with a resonance quality factor and/or a resonant RF frequency based on at least one characteristic of the at least one RF antenna. The RF antenna 53 device 20 may have any of the features or properties discussed above. The kit may also comprise a plurality of RF antennas 53, which the properties of the device being based on the one or more characteristics of the plurality of RF antennas as described above.

The device 20 of the kit 800 is the product of any of the methods 800 of producing the device for manipulating a magnetic field of RF radiation from one or more RF antenna in an MR system as described above. In particular, the device 20 is made in accordance with a design which provides the device with a target resonance quality factor and/or target resonant RF frequency of the device, which are determined based on at least one characteristic of the RF antenna 53. Hence the device 20 and RF antenna 53 are configured with complementary properties to provide improved performance of an MRI system using the kit 800.

The kit may further include the computer program product as describe above.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described but can be practised with modification and alteration within the scope of the appended claims. Accordingly, the specification and drawings are to be regarded in an illustrative sense rather than a restrictive sense. Although various features of the approach of the present disclosure have been presented separately (e.g., in separate figures), the skilled person will understand that, unless they are presented as mutually exclusive, they may each be combined with any other feature or combination of features of the present disclosure.

Aspects and features of the present disclosure are set forth in the following numbered clauses which contain the subject matter of the claims of the parent application as filed.

1. A method of producing a device for manipulating a magnetic field of RF radiation from one or more RF antenna in an MR system, comprising:

determining a target resonance quality factor of the device based on at least one characteristic of the one or more RF antenna; determining a design of the device to provide the device with the determined target resonance quality factor, wherein the device comprises:

a plurality of conductive elements arranged in an array, wherein the array is arranged to redistribute energy between electric and magnetic fields of the RF radiation at a resonant RF frequency when receiving the RF radiation, the RF radiation having an RF wavelength greater than a respective dimension of each conductive element; and a dielectric material, wherein the dielectric material has a dielectric permittivity and a loss tangent; and

making the device in accordance with the design.

2. A method of producing a device for manipulating a magnetic field of RF radiation from one or more RF antenna in an MR system, comprising:

determining a target resonant RF frequency of the device based on at least one characteristic of the one or more RF antenna; determining a design of the device to provide the device with the determined target resonant RF frequency of the device, wherein the device comprises:

a plurality of conductive elements arranged in an array, wherein the array is arranged to redistribute energy between electric and magnetic fields of the RF radiation at a resonant RF frequency when receiving the RF radiation, the RF radiation having an RF wavelength greater than a respective dimension of each conductive element; and a dielectric material, wherein the dielectric material has a dielectric permittivity and a loss tangent; and

making the device in accordance with the design.

3. The method of clause 1 or 2, wherein the design of the device includes one or more of: a positioning of the dielectric material relative to the plurality of conductive elements; the dielectric permittivity; the loss tangent; and one or more electronic components connected between two respective portions of the conductive elements.

4. The method of clause 3, wherein the design of the device includes the one or more electronic components and the device comprises a controller configured to control the one or more electronic components.

5. The method of any preceding clause, wherein each conductive element is elongate, wherein the dielectric material is located along a portion of each end of the conductive elements, wherein the length of the portions is selected based on the at least one characteristic of the one or more RF antenna.

6. The method of clause 5, wherein the length of the portion of each end of the conductive elements along which the dielectric material is located is determined based on the at least one characteristic of the one or more RF antenna.

7. The method of clause 5 or 6, wherein the device is arranged such that the length of the portion of each end of the conductive elements along which the dielectric material is located can be varied.

8. The method of clause 7, wherein the device is arranged such that the dielectric material is slidable in direction along the length of the conductive elements.

9. The method of any preceding clause, wherein the at least one characteristic of the one or more RF antenna includes one or more of: an RF frequency that the one or more RF antenna is arranged to transmit or receive; a resonance quality factor of the one or more RF antenna; and an object to be imaged by the one or more RF antenna.

10. The method of clause 9, wherein the loss tangent of the dielectric material is determined based on the resonance quality factor of the one or more RF antenna such that the resonance quality factor of the device is less than the resonance quality factor of the one or more RF antenna.

11. The method of clause 9 or 10, wherein the wherein the loss tangent of the dielectric material is determined based on the resonance quality factor of the one or more RF antenna in order that the resonance quality factor of the device is such that the device does not detune the one or more RF antenna when used in the MR system.

12. The method of any preceding clause, wherein the at least one characteristic of the one or more RF antenna includes at least one characteristic of a plurality of RF antennas.

13. The method of clause 12, wherein the loss tangent of the dielectric material is determined based on resonance quality factors of the plurality of RF antennas such that the resonance quality factor of the device is less than a lowest value of the resonance quality factors of the plurality of RF antennas.

14. The method according to any preceding clause, wherein the dielectric material is ceramic.

15. The method of any preceding clause, wherein the dielectric permittivity is greater than 50, optionally, greater than 100.

16. The method of any preceding clause, wherein the plurality of conductive elements is a plurality of metal strips on a printed circuit board, PCB.

17. The method of any preceding clause, wherein the device is a first device, the method further comprising:

making a second device in accordance with the design; and
assembling the first device and the second device on either side of one or more spacer located at at least one end of the conductive elements to provide an imaging region between the first device and second device, wherein the imaging region is for receiving an object to be imaged.

18. The method of any preceding clause, wherein the device is configured to concentrate a magnetic field of RF radiation from the one or more RF antenna when in the MR system.

19. A kit comprising:

one or more RF antenna having at least one characteristic; and

a device for manipulating a magnetic field of RF signals in an MR system, the device comprising:

a plurality of conductive elements arranged in an array, wherein the array is arranged to redistribute energy between electric and magnetic fields of the RF radiation at a resonant RF frequency when receiving the RF radiation, the RF radiation having an RF wavelength greater than a respective dimension of each conductive element; and
a dielectric material, wherein the dielectric material has a dielectric permittivity and a loss tangent;
wherein a device is arranged to resonate with a resonance quality factor and/or a resonant RF frequency based on the at least one characteristic of the one or more RF antenna.

20. The kit of clause 19, wherein the at least one characteristic is a resonance quality factor of the one or more RF antenna and the resonance quality factor of the device is less than the resonance quality factor of the one or more RF antenna.

21. The kit of clause 19 or 20, wherein the device is made using the method of any of clauses 1 to 18.

22. An MR system comprising:

an imaging region arranged to receive an object to be imaged;
a magnetic field generator arranged to produce a static magnetic field in the imaging region;
the kit of any of clauses 19 to 21, wherein the one or more RF antenna is arranged to irradiate the object with the RF radiation; and
an RF receiver arranged to receive a return RF signal from the object for imaging the object;
wherein the device is arranged between the imaging region and either the one or more RF antenna or the RF receiver, or both.

**Claims**

1. A method of producing a device for manipulating a magnetic field of RF radiation from one or more RF antenna in an MR system, comprising:

determining a target resonant RF frequency of the device based on at least one characteristic of the one or more RF antenna;
determining a design of the device to provide the device with the determined target resonant RF frequency, the device comprising:

a plurality of conductive elements arranged in an array to redistribute energy between electric and magnetic fields of the RF radiation at a resonant RF frequency when receiving the RF radiation, the RF radiation having an RF wavelength greater than a respective dimension of each conductive element; and
a dielectric material having a dielectric permittivity and a loss coefficient; and

making the device in accordance with the design.

2. The method of claim 1, further comprising: determining a target resonance quality factor of the device based on at least one characteristic of the one or more RF antenna, the design providing the device with the determined target resonance quality factor.

3. The method of claim 1 or 2, wherein the design of the device includes one or more of:

a positioning of the dielectric material relative to the plurality of conductive elements;
the dielectric permittivity; and
the loss coefficient.

4. The method of any preceding claim, wherein the design of the device includes one or more electronic components connected between two respective portions of the conductive elements.

5. The method of any preceding claim, wherein:

each conductive element is elongate;
the dielectric material is located along a portion of each end of the conductive elements; and
a length of the portions is selected based on the at least one characteristic of the one or more RF antenna.

6. The method of claim 5, wherein the device is arranged such that the length of the portion of each end of the conductive elements along which the dielectric material is located can be varied.

7. The method of claim 6, wherein the device is arranged such that the dielectric material is slidable in direction along the length of the conductive elements.

8. The method of any preceding claim, wherein the at least one characteristic of the one or more RF antenna includes one or more of:

an RF frequency that the one or more RF antenna is arranged to transmit or receive; a resonance quality factor of the one or more RF antenna; and
an object to be imaged by the one or more RF antenna.

9. The method of any preceding claim, wherein the at least one characteristic of the one or more RF antenna includes at least one characteristic of a plurality of RF antennas.

10. The method of any preceding claim, wherein the relative dielectric permittivity is greater than 50 or greater than 100.

11. The method of any preceding claim, wherein the device is a first device, the method further comprising:

making a second device in accordance with the design; and
assembling the first device and the second device on either side of one or more spacer located at at least one end of the conductive elements to provide an imaging region between the first device and second device, wherein the imaging region is for receiving an object to be imaged.

12. A kit comprising:

one or more RF antenna having at least one characteristic; and
a device for manipulating a magnetic field of RF signals in an MR system, the device comprising:

a plurality of conductive elements arranged in an array to redistribute energy between electric and magnetic fields of the RF radiation at a resonant RF frequency when receiving the RF radiation, the RF radiation having an RF wavelength greater than a respective dimension of each conductive element; and
a dielectric material having a dielectric permittivity and a loss coefficient;

the device being arranged to resonate with a resonant RF frequency based on the at least one characteristic of the one or more RF antenna.

13. The method of claim 12, wherein:

each conductive element is elongate;
the dielectric material is located along a portion of each end of the conductive elements; and
the device is arranged such that a length of the portion of each end of the conductive elements along which the dielectric material is located can be varied.

14. The method of claim 13, wherein the device is arranged such that the dielectric material is slidable in direction along the length of the conductive elements.

15. An MR system comprising:

an imaging region arranged to receive an object to be imaged;
a magnetic field generator arranged to produce a static magnetic field in the imaging region;
the kit of claim 12 to 14, the one or more RF antenna being arranged to irradiate the object with the RF radiation; and
an RF receiver arranged to receive a return RF signal from the object for imaging the object;

the device being arranged between the imaging region and either the one or more RF antenna or the RF receiver, or both.

Fig. 1

Fig. 2

Fig. 3

Fig. 4A

40

20

42    44    42

20

**Fig. 4B**

40

20

42    44    42

20

**Fig. 4C**

Fig. 5

Low power no MS & Frame no-23

Low power no MS & Frame no-76

Fig. 6

700

702

Determining a target resonance quality
factor and/or a target resonant RF
frequency

704

Determining a design of the device to
provide the device with the determined
target resonance quality factor and/or
target resonant RF frequency

706

Making the device in accordance with the
design

Fig. 7

800

53

20

Fig. 8

**EUROPEAN SEARCH REPORT**

Application Number

EP 23 17 0840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 318 188 A1 (SAINT PETERSBURG NATIONAL RESEARCH UNIV OF INFORMATION TECHNOLOGIES ME) 9 May 2018 (2018-05-09) | 1,3,5, 8-13,15 | INV. G01R33/28 G01R33/34 |
| A | * paragraph [0010] - paragraph [0025] * | 2 | H01Q15/00 |
| X | KRETOV EGOR I ET AL: "Control of the magnetic near-field pattern inside MRI machine with tunable metasurface", APPLIED PHYSICS LETTERS, A I P PUBLISHING LLC, US, vol. 115, no. 6, 8 August 2019 (2019-08-08), XP012239720, ISSN: 0003-6951, DOI: 10.1063/1.5099413 [retrieved on 2019-08-08] | 1,3,5-9, 11-15 | |
| A | * figure 2 * * abstract * * page 2 - page 3 * | 2 | |
| X | SHIMUL CHANDRA SAHA ET AL.: "A switch matrix to enable passive cloaking of a metasurface resonator for MRI applications", PROCEEDINGS OF THE INTERNATIONAL SOCIETY FOR MAGNETIC RESONANCE IN MEDICINE, ISMRM, 27TH ANNUAL MEETING AND EXHIBITION, MONTRÉAL, QUÉBEC, CANADA, 11 MAY - 16 MAY 2019, no. 1563, 26 April 2019 (2019-04-26), XP040708949, | 1,3,4, 8-12,15 | TECHNICAL FIELDS SEARCHED (IPC) G01R H01Q |
| A | * the whole document * | 2 | |

-/--

The present search report has been drawn up for all claims

| Place of search | Date of completion of the search | Examiner |
|---|---|---|
| Munich | 3 July 2023 | Durst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 23 17 0840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALENA SHCHELOKOVA ET AL.: "Demonstration of a new volumetric wireless coil for extremities imaging", PROCEEDINGS OF THE INTERNATIONAL SOCIETY FOR MAGNETIC RESONANCE IN MEDICINE, ISMRM, JOINT ANNUAL MEETING ISMRM-ESMRMB, PARIS, FRANCE, 16-21 JUNE 2018, no. 26, 1 June 2018 (2018-06-01), XP040699235, | 1,3,8,9, 12,15 | |
| A | * the whole document * | 2 | |

-----

TECHNICAL FIELDS
SEARCHED        (IPC)

The present search report has been drawn up for all claims

| Place of search | Date of completion of the search | Examiner |
|---|---|---|
| Munich | 3 July 2023 | Durst, Markus |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

**ANNEX TO THE EUROPEAN SEARCH REPORT**

**ON EUROPEAN PATENT APPLICATION NO.**                    EP 23 17 0840

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2023

| Patent document cited in search report | | Publication date | Patent family member(s) | | Publication date |
|---|---|---|---|---|---|
| EP 3318188 | A1 | 09-05-2018 | CN | 107835658 A | 23-03-2018 |
| | | | EA | 201792585 A1 | 30-04-2018 |
| | | | EP | 3318188 A1 | 09-05-2018 |
| | | | ES | 2869906 T3 | 26-10-2021 |
| | | | JP | 6770537 B2 | 14-10-2020 |
| | | | JP | 2018520777 A | 02-08-2018 |
| | | | KR | 20180040572 A | 20-04-2018 |
| | | | RU | 2601373 C1 | 10-11-2016 |
| | | | US | 2018188339 A1 | 05-07-2018 |
| | | | WO | 2017007365 A1 | 12-01-2017 |

EPO FORM P0459

For more details about this annex : see Official Journal of the European Patent Office, No. 12/82

**REFERENCES CITED IN THE DESCRIPTION**

**Patent documents cited in the description**

- WO 2017007365 A